# EUROPEAN PATENT APPLICATION

(11) **EP 3 896 175 A1**
(43) Date of publication of application: **20.10.2021**
(21) Application number: 19897412.3
(22) Date of filing: 20.11.2019
(51) Int. Cl.: C12Q 1/689, A23L 33/135, A61K 8/99, A61K 9/00, A61K 35/74, A61P 29/00, A61Q 19/00

(54) **NANO-VESICLE DERIVED FROM CORYNEBACTERIUM SP. BACTERIA AND USE THEREOF**

(30) Priority: 10.12.2018 KR 20180158622; 23.10.2019 KR 20190132137
(71) Applicant: MD Healthcare Inc., Seoul 03923 (KR)
(72) Inventor: KIM, Yoon-Keun, Paju-Si, Gyeonggi-do 10908 (KR)
(74) Representative: Dehns
(86) International application number: PCT/KR2019/015901
(87) International publication number: WO 2020/122449

(57) **Abstract**

The invention relates to vesicles derived from bacteria of the genus *Corynebacterium* and a use thereof. The present inventors experimentally identified that vesicles derived from bacteria of the genus *Cornynebacterium* were in reduced levels in clinical samples of patients with cirrhosis, stroke, diabetes, asthma, atopic dermatitis, depression, breast cancer, dementia, and nasal polyps, and effectively suppress the secretion of inflammatory mediators from pathogenic vesicles inducing inflammation. Thus, it is expected that the vesicles derived from bacteria of the genus *Cornynebacterium* according to the present invention will be advantageously used as a composition for prevention or treatment of inflammatory diseases comprising cirrhosis, stroke, diabetes, asthma, atopic dermatitis, depression, breast cancer, dementia, nasal polyps, and the like.

## Description

### [Technical Field]

The present invention relates to nano-vesicles derived from bacteria of the genus *Corynebacterium* and a use thereof, and more particularly to a method for diagnosing cirrhosis, stroke, diabetes, asthma, atopic dermatitis, depression, breast cancer, dementia, and nasal polyps using nano-vesicles derived from bacteria of the genus *Corynebacterium,* a composition for prevention, alleviation, or treatment of the disease, comprising the vesicles, and the like.

This application claims priority to and the benefit of Korean Patent Application Nos. 10-2018-0158622 and 10-2019-0132137 filed in the Korean Intellectual Property Office on December 10, 2018 and October 23, 2019, respectively, and all the contents disclosed in the specification and drawings of the applications are incorporated in this application.

### [Background Art]

Since the beginning of the 21st century, acute infectious diseases recognized as epidemic diseases in the past have become less important, whereas chronic inflammatory diseases accompanied by immune dysfunction caused by disharmony between humans and microbiomes have changed disease patterns as main diseases. In particular, as chronic inflammatory diseases such as obesity, diabetes, cardiovascular disease, neuro-psychiatric disorders, and cancer due to westernized eating habits, indoor air pollution due to changes in house structure, and indoor living time are increased, skin and respiratory inflammatory diseases have become a major health problem for people.

The development of the inflammatory diseases is accompanied by abnormalities in the immune function against external causative factors. The Th17 immune response, which secretes the interleukin (hereinafter, referred to as IL)-17 cytokine, is important for the immune response to causative factors derived from bacteria, and neutrophil inflammation due to the Th17 immune response occurs upon exposure to bacterial causative factors. Further, inflammatory mediators such as tumor necrosis factor-alpha (hereinafter, referred to as TNF-α), which is secreted by bacterial causative factors during the development of inflammation, play an important role in inflammation and cancer development. It has been recently reported that among inflammatory mediators, IL-6, which is secreted by bacterial causative factors, plays an important role in differentiation into Th17 cells, and chronic inflammation caused by the Th17 immune response is closely related to the development of chronic inflammatory diseases as well as cancer.

It is known that the number of microorganisms coexisting in the human body has reached 100 trillion, which is 10 times more than the number of human cells, and the number of microorganism genes is more than 100 times the number of human genes. A microbiota or microbiome refers to a microbial community including bacteria, archaea, and eukarya which are present in a given habitat, and it is known that the gut microbiota or microbiome plays an important role in human physiological phenomena and has a great influence on human health and disease through interaction with human cells.

Bacteria and archaea coexisting in our body secrete nanometer-sized vesicles in order to exchange information on genes, proteins, and the like with other cells. The mucosa forms a physical defense membrane through which particles having a size of 200 nanometers (nm) or more cannot pass, so that bacteria coexisting in the mucosa cannot pass through the mucosa, but vesicles derived from bacteria have a size of 100 nanometers or less and are absorbed into our bodies after relatively freely passing through epithelial cells via the mucosa. It has recently been revealed that pathogenic bacteria-derived vesicles absorbed in our body play an important role in the pathogenesis of metabolic diseases such as diabetes and obesity.

Bacteria of the genus *Corynebacterium* are aerobic gram-positive bacteria, and known as bacteria that are widely spread in nature and live in symbiosis with eukaryotes. In particular, *Corynebacterium glutamicum* bacteria are bacteria that are widely used industrially for the production of amino acids, nucleic acids, and the like. However, there is still no report on a treatment technique using extracellular vesicles of bacteria of the genus *Cornynebacterium.*

Thus, in the present invention, it was confirmed that by isolating vesicles from bacteria of the genus *Cornynebacterium* for the first time and confirming the characteristics thereof, the vesicles could be used as a composition for diagnosis of various inflammatory diseases and for prevention, alleviation, or treatment of the inflammatory diseases.

### [Disclosure]

### [Technical Problem]

As a result of conducting earnest research to solve the above conventional problems, the inventors confirmed that a content of vesicles derived from bacteria of the genus *Cornynebacterium* is significantly decreased in a sample derived from a patient with cirrhosis, stroke, diabetes, asthma, atopic dermatitis, depression, breast cancer, dementia, and nasal polyps, compared with a normal individual, through metagenomic analysis. Further, the present inventors confirmed that vesicles derived from bacteria of the genus *Cornynebacterium* efficiently suppress the inflammatory response caused by pathogenic vesicles, thereby completing the present invention based on this.

Thus, an object of the present invention is to provide a method of diagnosing one or more diseases selected from the group consisting of cirrhosis, stroke, diabetes, asthma, atopic dermatitis, depression, breast cancer, dementia, and nasal polyps, or a method of providing information for diagnosis thereof.

Further, another object of the present invention is to provide a composition for preventing, alleviating, or treating an inflammatory disease, comprising vesicles derived from bacteria of the genus *Cornynebacterium* as an active ingredient.

However, a technical problem to be achieved by the present invention is not limited to the aforementioned problems, and the other problems that are not mentioned may be clearly understood by a person skilled in the art from the following description.

### [Technical Solution]

To achieve the object of the present invention as described above, the present invention provides a method of providing information for diagnosing one or more diseases selected from the group consisting of cirrhosis, stroke, diabetes, asthma, atopic dermatitis, depression, breast cancer, dementia, and nasal polyps, the method comprising the following steps:
(a) extracting DNAs from vesicles isolated from samples of a normal individual and a subj ect;
(b) performing polymerase chain reaction (PCR) on the extracted DNA using a pair of primers prepared based on a gene sequence present in 16S rDNA to obtain each PCR product; and
(c) determining a case in which a content of vesicles derived from bacteria of the genus *Cornynebacterium* is lower than that of the normal individual sample, as one or more diseases selected from the group consisting of cirrhosis, stroke, diabetes, asthma, atopic dermatitis, depression, breast cancer, dementia, and nasal polyps, through quantitative analysis of the PCR product.

In addition, the present invention provides a method of diagnosing one or more diseases selected from the group consisting of cirrhosis, stroke, diabetes, asthma, atopic dermatitis, depression, breast cancer, dementia, and nasal polyps, the method comprising the following steps:
(a) extracting DNAs from vesicles isolated from samples of a normal individual and a subj ect;
(b) performing polymerase chain reaction (PCR) on the extracted DNA using a pair of primers prepared based on a gene sequence present in 16S rDNA to obtain each PCR product; and
(c) determining a case in which a content of vesicles derived from bacteria of the genus *Cornynebacterium* is lower than that of the normal individual sample, as one or more diseases selected from the group consisting of cirrhosis, stroke, diabetes, asthma, atopic dermatitis, depression, breast cancer, dementia, and nasal polyps, through quantitative analysis of the PCR product.

As an exemplary embodiment of the present invention, the sample in step (a) may be blood, urine, stool, saliva, or nasal mucosa.

As another embodiment of the present invention, the pair of primers in Step (b) may be a pair of primers comprising base sequences represented by SEQ ID Nos. 1 and 2.

Further, the present invention provides a composition for preventing, alleviating, or treating an inflammatory disease, comprising vesicles derived from bacteria of the genus *Cornynebacterium* as an active ingredient.

The composition may comprise a pharmaceutical composition, a food composition, a cosmetic composition, and an inhalable composition.

Further, the present invention provides a method of preventing or treating an inflammatory disease, the method comprising a step of administering a composition comprising vesicles derived from bacteria of the genus *Cornynebacterium* as an active ingredient to a subject.

Further, the present invention provides a use of vesicles derived from bacteria of the genus *Cornynebacterium* for preventing or treating an inflammatory disease.

Further, the present invention provides a use of a composition comprising vesicles derived from bacteria of the genus *Cornynebacterium* as an active ingredient for preventing or treating an inflammatory disease.

Further, the present invention provides a use of vesicles derived from bacteria of the genus *Cornynebacterium* for preparing a drug used for an inflammatory disease.

As an exemplary embodiment of the present invention, the vesicles may be secreted from a *Cornynebacterium glutamicum.*

As another exemplary embodiment of the present invention, the vesicles may have an average diameter of 10 to 200 nm.

As still another exemplary embodiment of the present invention, the vesicles may be secreted naturally or artificially from bacteria of the genus *Cornynebacterium.*

As yet another embodiment of the present invention, the artificially secreted vesicles may be secreted by performing a method such as heat treatment and pressure treatment on the bacteria.

As yet another exemplary embodiment of the present invention, the inflammatory disease may be one or more diseases selected from the group consisting of cirrhosis, stroke, diabetes, asthma, atopic dermatitis, depression, breast cancer, dementia, and nasal polyps.

### [Advantageous Effects]

The present inventors found that bacteria were not absorbed in the body, but vesicles derived from bacteria passed through the protective membrane of the mucosa, and were absorbed by the mucosal epithelial cells, systemically distributed, and excreted from the body through the kidneys, liver, and lungs. Further, through a metagenomic analysis of vesicles derived from bacteria present in a patient's blood, it was identified that vesicles derived from bacteria of the genus *Cornynebacterium* present in the blood or nasal mucosa of patients with cirrhosis, stroke, diabetes, asthma, atopic dermatitis, depression, breast cancer, dementia, and nasal polyps were significantly reduced compared to normal individual. In addition, it was found that when *Corynebacterium glutamicum,* which is a species of bacteria of the genus *Corynebacterium,* was cultured *ex vivo* and vesicles were separated and administered to inflammatory cells, the secretion of inflammatory mediators such as TNF-α from pathogenic vesicles were significantly suppressed, so that it is expected that the vesicles derived from bacteria of the genus *Corynebacterium* according to the present invention will be advantageously used for a composition for prevention or treatment of the inflammatory diseases.

### [Description of Drawings]

FIG. 1A is a series of photographs capturing distribution patterns of bacteria and bacteria-derived vesicles (EV) by time after the bacteria and the vesicles derived from bacteria were orally administered to mice, and FIG. 1B is a result of evaluating the in vivo distribution patterns of the bacteria and the vesicles by harvesting blood, kidneys, liver, and various organs at 12 hours after orally administering the bacteria and the vesicles.
FIG. 2 is a view of evaluating whether bacteria and bacteria-derived vesicles (EV) are infiltrated into mucosal epithelial cells after administering the bacteria and bacteria-derived vesicles to a mouse (Lu, lumen; LP, lamina propria).
FIG. 3 is a result of comparing the distributions of vesicles derived from bacteria of the genus *Corynebacterium* after metagenomic analysis of vesicles derived from bacteria present in the blood of cirrhosis patients and normal individuals.
FIG. 4 is a result of comparing the distributions of vesicles derived from bacteria of the genus *Corynebacterium* after metagenomic analysis of vesicles derived from bacteria present in the blood of stroke patients and normal individuals.
FIG. 5 is a result of comparing the distributions of vesicles derived from bacteria of the genus *Corynebacterium* after metagenomic analysis of vesicles derived from bacteria present in the blood of diabetes patients and normal individuals.
FIG. 6 is a result of comparing the distributions of vesicles derived from bacteria of the genus *Corynebacterium* after metagenomic analysis of vesicles derived from bacteria present in the blood of asthma patients and normal individuals.
FIG. 7 is a result of comparing the distributions of vesicles derived from bacteria of the genus *Corynebacterium* after metagenomic analysis of vesicles derived from bacteria present in the blood of atopic dermatitis patients and normal individuals.
FIG. 8 is a result of comparing the distributions of vesicles derived from bacteria of the genus *Corynebacterium* after metagenomic analysis of vesicles derived from bacteria present in the blood of depression patients and normal individuals.
FIG. 9 is a result of comparing the distributions of vesicles derived from bacteria of the genus *Corynebacterium* after metagenomic analysis of vesicles derived from bacteria present in the blood of breast cancer patients and normal individuals.
FIG. 10 is a result of comparing the distributions of vesicles derived from bacteria of the genus *Corynebacterium* after metagenomic analysis of vesicles derived from bacteria present in the blood of dementia patients and normal individuals.
FIG. 11 is a view in which the infiltration patterns of vesicles derived from bacteria of the genus *Corynebacterium* were evaluated in the nasal mucosal tissues of allergic and nonallergic nasal polyps patients and a normal control through metagenomic analysis.
FIG. 12 is a result of evaluating apoptosis by treating microphages (Raw264.7 cells) with vesicles derived from *Corynebacterium glutamicum* in order to evaluate the apoptotic effects of vesicles derived from *Corynebacterium glutamicum* (EV, extracellular vesicle; CGT101, *Corynebacterium glutamicum* EV).
FIGS. 13A and 13B are results of comparing the secretion level of inflammatory mediators with that of *E. coli* EV which is a pathogenic vesicle by treating macrophages (Raw264.7 cells) with vesicles (CGT101) derived from *Corynebacterium glutamicum* in order to evaluate the inflammation induction effects of vesicles derived from *Corynebacterium glutamicum,* FIG. 13A compares the secretion levels of IL-6, and FIG. 13B compares the secretion levels of TNF-α (EV: extracellular vesicle).
FIG. 14 is a results of evaluating an effect on the secretion of TNF-α, which is an inflammatory mediator, from *E. coli* EVs by pre-treating vesicles derived from bacteria of the genus *Corynebacterium* prior to the treatment of *E. coli* EVs which is a pathogenic vesicle in order to evaluate the anti-inflammatory effects of vesicles (CGT101) derived from *Corynebacterium glutamicum* (EV, extracellular vesicle).

### [Modes of the Invention]

The present invention relates to vesicles derived from bacteria of the genus *Corynebacterium* and a use thereof.

The present inventors confirmed through a metagenomic analysis that the content of vesicles derived from bacteria of the genus *Cornynebacterium* in samples derived from patients with cirrhosis, stroke, diabetes, asthma, atopic dermatitis, depression, breast cancer, dementia, and nasal polyps was remarkably reduced compared to normal individuals. Further, the present inventors confirmed that the inflammatory response caused by pathogenic causative factors was efficiently suppressed by treating inflammatory cells with vesicles derived from *Corynebacterium glutamicum* bacterium before administering the pathogenic causative factors, thereby completing the present invention based on this.

Thus, the present invention provides a method of diagnosing one or more diseases selected from the group consisting of cirrhosis, stroke, diabetes, asthma, atopic dermatitis, depression, breast cancer, dementia, and nasal polyps, or a method of providing information for diagnosis thereof, the method comprising the following steps:
(a) extracting DNAs from vesicles isolated from samples of a normal individual and a subj ect;
(b) performing polymerase chain reaction (PCR) on the extracted DNA using a pair of primers prepared based on a gene sequence present in 16S rDNA to obtain each PCR product; and
(c) determining a case in which a content of vesicles derived from bacteria of the genus *Cornynebacterium* is lower than that of the normal individual sample, as one or more diseases selected from the group consisting of cirrhosis, stroke, diabetes, asthma, atopic dermatitis, depression, breast cancer, dementia, and nasal polyps, through quantitative analysis of the PCR product.

The term "diagnosis" as used herein refers to determination of a condition of a disease of a patient over all aspects, in a broad sense. The contents of the determination are the disease entity, the etiology, the pathogenesis, the severity, the detailed aspects of a disease, the presence and absence of complications, the prognosis, and the like. The diagnosis in the present invention means determining whether one or more diseases selected from the group consisting of cirrhosis, stroke, diabetes, asthma, atopic dermatitis, depression, breast cancer, dementia, and nasal polyps occur, the level of the disease, and the like.

The term "nanovesicle" or "vesicle" as used herein refers to a structure consisting of a nano-sized membrane secreted from various bacteria. Vesicles derived from gram-positive bacteria such as *Corynebacterium* include peptidoglycan and lipoteichoic acid, which are constituents of bacterial cell walls, and various low-molecular weight compounds in the vesicles, in addition to proteins and nucleic acids. In the present invention, nanovesicles or vesicles are secreted naturally from bacteria of the genus *Corynebacterium* or produced artificially by performing heat treatment, pressure treatment, or the like on the bacteria, and have an average diameter of 10 to 200 nm.

The term "metagenome" as used herein also refers to a microbiome, and refers to a total of genomes including all viruses, bacteria, fungi, and the like in an isolated region such as soil and an animal's intestines, and is typically used as a concept of genomes explaining identification of a large number of microorganisms at one time by using a sequence analyzer in order to analyze uncultivated microorganisms. In particular, the metagenome does not refer to a genome of one species, but refers to a kind of mixed genome as a genome of all species of one environmental unit. The metagenome is, when one species is defined in the development process of omics biology, a term derived from the viewpoint of making a complete species is made by various species interacting with each other as well as one kind of functionally existing species. Technically, the metagenome is an object of a technique to identify all species in one environment and investigate interactions and metabolism by analyzing all DNAs and RNAs regardless of species using a rapid sequence analysis method.

The vesicles may be isolated from a culturing solution comprising bacteria of the genus *Corynebacterium* by using one or more methods selected from the group consisting of centrifugation, ultra-high speed centrifugation, high pressure treatment, extrusion, sonication, cell lysis, homogenization, freezing-thawing, electroporation, mechanical decomposition, chemical treatment, filtration by a filter, gel filtration chromatography, free-flow electrophoresis, and capillary electrophoresis. Further, a process such as washing for removing impurities and concentration of obtained vesicles may be further included.

In the present invention, the sample in Step (a) may be blood, urine, stool, saliva, or nasal mucosa, but is not limited thereto.

In the present invention, the pair of primers in Step (b) may be a pair of primers comprising base sequences represented by SEQ ID Nos. 1 and 2, but is not limited thereto.

As another aspect of the present invention, the present invention provides a composition for preventing, alleviating, or treating an inflammatory disease, comprising vesicles derived from bacteria of the genus *Corynebacterium* as an active ingredient.

The composition comprises a pharmaceutical composition, a food composition, a cosmetic composition, and an inhalable composition.

As another aspect of the present invention, the present invention provides a method of preventing or treating an inflammatory disease, the method comprising a step of administering a composition comprising vesicles derived from bacteria of the genus *Cornynebacterium* as an active ingredient to a subject.

As another aspect of the present invention, the present invention provides a use of vesicles derived from bacteria of the genus *Cornynebacterium* for preventing or treating an inflammatory disease.

As another aspect of the present invention, the present invention provides a use of a composition comprising vesicles derived from bacteria of the genus *Cornynebacterium* as an active ingredient for preventing or treating an inflammatory disease.

As another aspect of the present invention, the present invention provides a use of vesicles derived from bacteria of the genus *Cornynebacterium* for preparing a drug used for an inflammatory disease.

The term "inflammatory disease" as used herein refers to a disease which is caused by damage to skin or intestinal epithelial cells and consequent inflammation as a result of exposure to causative factors that induce inflammation, and includes metabolic diseases, cardiovascular diseases, neuro-psychiatric diseases, and cancer which occur as a result of inflammation, but is not limited thereto.

Examples of the inflammatory disease according to the present invention include cirrhosis, stroke, diabetes, asthma, atopic dermatitis, depression, breast cancer, dementia, nasal polyps, and the like, but are not limited thereto.

The term "prevention" as used herein refers to all actions that suppress an inflammatory disease, or delay the onset thereof via administration of the composition according to the present invention.

The term "treatment" as used herein refers to all actions that alleviate or beneficially change symptoms of an inflammatory disease via administration of composition according to the present invention.

The term "alleviation" used as used herein refers to all actions that at least reduce a parameter associated with a condition to be treated, for example, the degree of symptoms.

In one embodiment of the present invention, as a result of orally administering bacteria and vesicles derived from bacteria to mice and observing in vivo absorption, distribution, and excretion patterns of the bacteria and the vesicles, it was confirmed that, while the bacteria were not absorbed via the intestinal mucous membrane, the vesicles derived from bacteria were absorbed within 5 minutes after administration and systemically distributed, and excreted via the kidneys, liver, and the like (see Example 1).

In another exemplary embodiment of the present invention, it was evaluated whether bacteria and vesicles derived from bacteria directly administered to the intestines passed through the protective membrane of the intestinal mucosa, and it was confirmed that bacteria failed to pass through the protective membrane of the intestinal mucosa, whereas vesicles derived from bacteria passed through the protective membrane of the mucosa. (See Example 2).

In still another exemplary embodiment of the present invention, through a metagenomic analysis of clinical samples of patients with cirrhosis, stroke, diabetes, asthma, atopic dermatitis, depression, breast cancer, dementia, and nasal polyps and a normal control, the distributions of bacteria of the genus *Corynebacterium* were compared, thus, it was confirmed that the distribution of vesicles derived from bacteria of the genus *Corynebacterium* in the clinical samples of the patients was significantly reduced compared to the normal control (see Examples 3 to 12).

In yet another exemplary embodiment of the present invention, inflammation induction effects of vesicles secreted from *Corynebacterium glutamicum* strains belonging to bacteria of the genus *Corynebacterium* were evaluated by culturing the strains, and as a result of comparing the secretion levels of inflammatory mediators by treating macrophages with the vesicles derived from *Corynebacterium glutamicum* at various concentrations, and then treating the macrophages with the *E. coli*-derived vesicles, which are pathogenic vesicles, the ability of inflammatory mediators to be secreted was remarkably reduced by the vesicles derived from *Corynebacterium glutamicum* as compared to the secretion of IL-6 and TNF-α by *E. coli*-derived vesicles (see Example 14).

In yet another exemplary embodiment of the present invention, anti-inflammatory effects of vesicles derived from *Corynebacterium glutamicum* strains were evaluated. As a result of evaluating the secretion of inflammatory mediators after treating macrophages with vesicles derived from *Corynebacterium glutamicum* at various concentrations prior to treatment with *E. coli*-derived vesicles, which are pathogenic vesicles, it was confirmed the vesicles derived from *Corynebacterium glutamicum* efficiently suppressed the secretion of TNF-α by inflammation-inducing *E. coli*-derived vesicles (see Examples 15).

The pharmaceutical composition of the present invention may include a pharmaceutically acceptable carrier. The pharmaceutically acceptable carrier is typically used in formulation, and includes saline, sterile water, Ringer's solution, buffered saline, cyclodextrin, a dextrose solution, a maltodextrin solution, glycerol, ethanol, liposomes, and the like, but is not limited thereto, and may further include other typical additives such as an antioxidant and a buffer, if necessary. Further, the composition may be formulated into an injectable formulation, such as an aqueous solution, a suspension, and an emulsion, a pill, a capsule, a granule, or a tablet by additionally adding a diluent, a dispersant, a surfactant, a binder, a lubricant, and the like. With regard to suitable pharmaceutically acceptable carriers and formulations, the composition may be preferably formulated according to each ingredient by using the method disclosed in the Remington's literature. The pharmaceutical composition of the present invention is not particularly limited in formulation, but may be formulated into an injection, an inhalant, an external preparation for skin, an oral ingestion, or the like.

The pharmaceutical composition of the present invention may be orally administered or may be parenterally administered (for example, administered intravenously, subcutaneously, intradermally, intranasally or intratracheally) according to the target method, and the administration dose may vary depending on the patient's condition and body weight, severity of disease, drug form, and administration route and period, but may be appropriately selected by those of ordinary skill in the art.

The pharmaceutical composition according to the present invention is administered in a pharmaceutically effective amount. In the present invention, the pharmaceutically effective amount refers to an amount sufficient to treat diseases at a reasonable benefit/risk ratio applicable to medical treatment, and an effective dosage level may be determined according to factors including types of diseases of patients, the severity of disease, the activity of drugs, sensitivity to drugs, administration time, administration route, excretion rate, treatment period, and simultaneously used drugs, and factors well known in other medical fields. The composition according to the present invention may be administered as an individual therapeutic agent or in combination with other therapeutic agents, may be administered sequentially or simultaneously with therapeutic agents in the related art, and may be administered in a single dose or multiple doses. It is important to administer the composition in a minimum amount that can obtain the maximum effect without any side effects, in consideration of all the aforementioned factors, and this may be easily determined by those of ordinary skill in the art.

Specifically, an effective amount of the pharmaceutical composition according to the present invention may vary depending on the age, sex, and body weight of a patient, and may be increased or decreased depending on the route of administration, severity of obesity, sex, body weight, age, and the like.

The inhalant composition of the present invention may be used by adding the active ingredient as it is to the inhalant, or may be used together with other ingredients, and may be appropriately used by a typical method. The mixing amount of active ingredient may be suitably determined depending on the purpose of use (for prevention or treatment).

The food composition of the present invention includes a health functional food composition. The food composition according to the present invention may be used by adding an active ingredient as is to food or may be used together with other foods or food ingredients, but may be appropriately used according to a typical method. The mixed amount of the active ingredient may be suitably determined depending on the purpose of use thereof (for prevention or alleviation). In general, when a food or beverage is prepared, the composition of the present invention is added in an amount of 15 wt% or less, preferably 10 wt% or less based on the raw materials. However, for long-term intake for the purpose of health and hygiene or for the purpose of health control, the amount may be less than the above-mentioned range.

Other ingredients are not particularly limited, except that the food composition of the present invention contains the active ingredient as an essential ingredient at the indicated ratio, and the food composition of the present invention may contain various flavorants, natural carbohydrates, and the like, like a typical beverage, as an additional ingredient. Examples of the above-described natural carbohydrate include common sugars such as monosaccharides, for example, glucose, fructose and the like; disaccharides, for example, maltose, sucrose and the like; and polysaccharides, for example, dextrin, cyclodextrin and the like, and sugar alcohols such as xylitol, sorbitol, and erythritol. As the flavorant other than those described above, a natural flavorant (thaumatin, stevia extract (for example, rebaudioside A, glycyrrhizin and the like), and a synthetic flavorant (saccharin, aspartame and the like) may be advantageously used. The proportion of the natural carbohydrate may be appropriately determined by the choice of those of ordinary skill in the art.

The food composition of the present invention may contain various nutrients, vitamins, minerals (electrolytes), flavoring agents such as synthetic flavoring agents and natural flavoring agents, colorants and fillers (cheese, chocolate, and the like), pectic acid and salts thereof, alginic acid and salts thereof, organic acids, protective colloid thickeners, pH adjusting agents, stabilizers, preservatives, glycerin, alcohols, carbonating agents used in a carbonated beverage, or the like, in addition to the additives. These ingredients may be used either alone or in combinations thereof. The ratio of these additives may also be appropriately selected by those of ordinary skill in the art.

The cosmetic composition of the present invention may comprise not only vesicles derived from bacteria of the genus *Corynebacterium,* but also ingredients commonly used in cosmetic compositions, and may comprise, for example, general adjuvants such as an antioxidant, a stabilizer, a solubilizing agent, vitamins, pigments, and herbs, and a carrier.

In addition, the composition of the present invention may further include, in addition to the vesicles derived from bacteria of the genus *Corynebacterium,* a mixture of organic UV blocking agents that have long been used within a range that does not adversely affect a skin protective effect by reaction with vesicles derived from bacteria of the genus *Corynebacterium.* The organic UV blocking agent may be one or more selected from the group consisting of glyceryl PABA, drometrizole trisiloxane, drometrizole, digalloyl trioleate, disodium phenyl dibenzimidazole tetrasulfonate, diethylhexyl butamido triazone, diethylamino hydroxy benzoyl hexyl benzoate, DEA-methoxycinnamate, a mixture of lawsone and dihydroxyacetone, methylenebis-benzotriazolyltetramethylbutylphenol, 4-methylbenzylidene camphor, menthyl anthranilate, benzophenone-3(oxybenzone), benzophenone-4, benzophenone-8(dioxybenzone), butylmethoxydibenzoylmethane, bisethylhexyloxyphenolmethoxyphenyltriazine, cinoxate, ethyldihydroxypropyl PABA, octocrylene, ethylhexyldimethyl PABA, ethylhexylmethoxycinnamate, ethylhexyl salicylate, ethylhexyl triazone, isoamyl-p-methoxycinnamate, polysilicon-15(dimethicodiethylbenzal malonate), terephthalylidene dicamphor sulfonic acid and salts thereof, TEA-salicylate, and para-aminobenzoic acid (PABA).

Examples of products to which the cosmetic composition of the present invention may be added include cosmetics such as astringents, skin softeners, nourishing toners, various creams, essences, packs, foundations, and the like, cleansings, face cleansers, soaps, treatments, beauty liquids, and the like. Particular preparations of the cosmetic composition of the present invention include a skin lotion, a skin softener, a skin toner, an astringent, a lotion, a milk lotion, a moisturizing lotion, a nourishing lotion, a massage cream, a nourishing cream, a moisturizing cream, a hand cream, an essence, a nourishing essence, a pack, a soap, a shampoo, a cleansing foam, a cleansing lotion, a cleansing cream, a body lotion, a body cleanser, an emulsion, a lipstick, a makeup base, a foundation, a press powder, a loose powder, an eye shadow, and the like.

Hereinafter, preferred Examples for helping the understanding of the present invention will be suggested. However, the following Examples are provided only to more easily understand the present invention, and the contents of the present invention are not limited by the following Examples.

### [Examples]

### Example 1. Analysis of in vivo Absorption, Distribution, and Excretion Patterns of Bacteria and Vesicles Derived from Bacteria

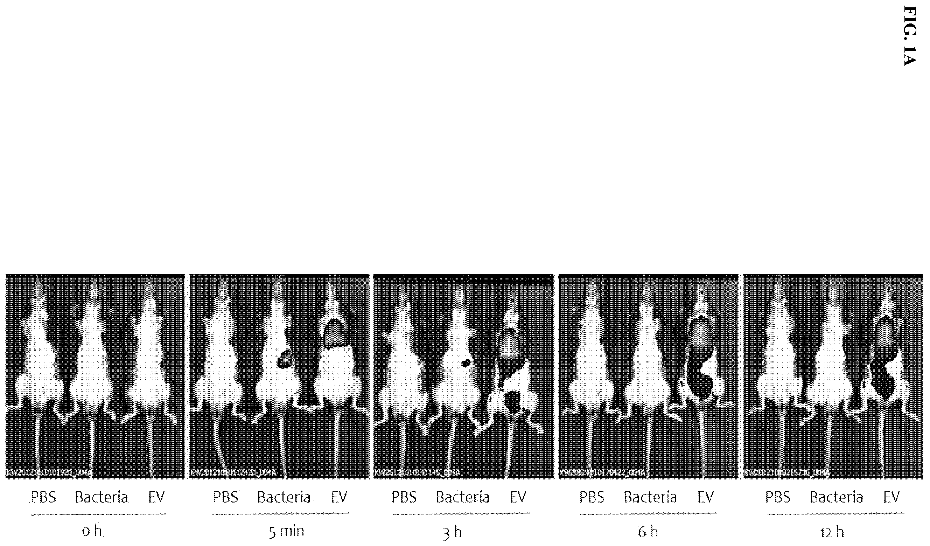

In order to evaluate whether bacteria and vesicles derived from bacteria were systemically absorbed through the mucosa, an experiment was performed as follows. A dose of 50 µg of each of fluorescence-labeled bacteria and vesicles derived from bacteria in the stomach of a mouse was administered to the gastrointestinal tract, and fluorescence was measured after 0 minute, 5 minutes, 3 hours, 6 hours, and 12 hours. As a result of observing the entire image of the mouse, as illustrated in FIG. 1A, the bacteria were not systemically absorbed, but the vesicles derived from bacteria were systemically absorbed 5 minutes after administration, and fluorescence was strongly observed in the bladder 3 hours after administration, so that it could be seen that the vesicles were excreted to the urinary tract. Further, it could be seen that the vesicles were present in the body until 12 hours after administration (see FIG. 1A).

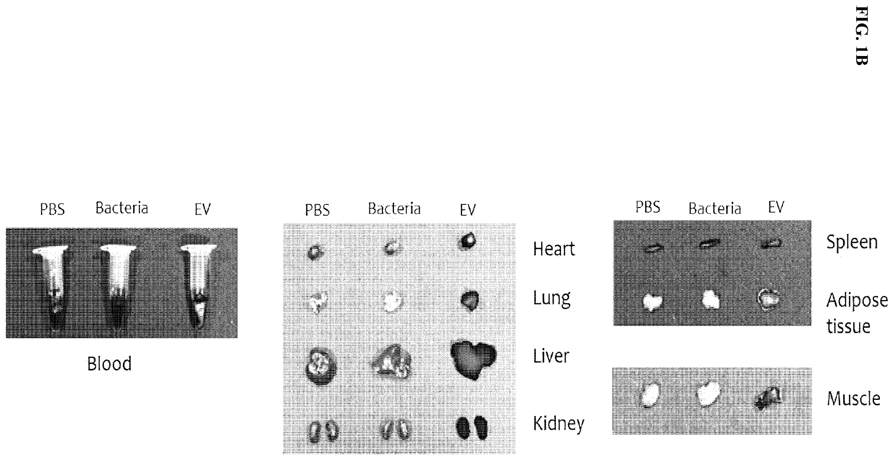

In order to evaluate the pattern in which the bacteria and the vesicles derived from the bacteria infiltrated into various organs after they were systemically absorbed, 50 µg of bacteria and vesicles derived from bacteria labeled with fluorescence were administered in the same manner as described above, and then the blood, heart, lungs, liver, kidneys, spleen, fat, and muscle were collected 12 hours after administration. As a result of observing fluorescence in the collected tissues, as illustrated in FIG. 1B, it could be seen that the vesicles derived from bacteria were distributed in the blood, heart, lungs, liver, kidneys, spleen, fat, and muscle but the bacteria were not absorbed (see FIG. 1B).

### Example 2. Evaluation of whether bacteria and vesicles derived from bacteria penetrate protective membrane of mucosa

In order to evaluate whether bacteria and bacteria-derived vesicles passed through the protective membrane of the mucosa to be infiltrated into epithelial tissue, after bacteria and bacteria-derived vesicles were directly administered to the intestines, infiltration into the epithelial tissue after passing through the protective membrane of the mucosa was evaluated by an immunohistochemistry method. In order to evaluate the presence of bacteria and vesicles in the mucosal tissue, antibodies against the bacteria and the vesicles were prepared, attached to a green fluorescent protein (GFP) and used, and after staining with 4, 6-diamidino 2-phenylindole (DAPI), observed under a microscope.

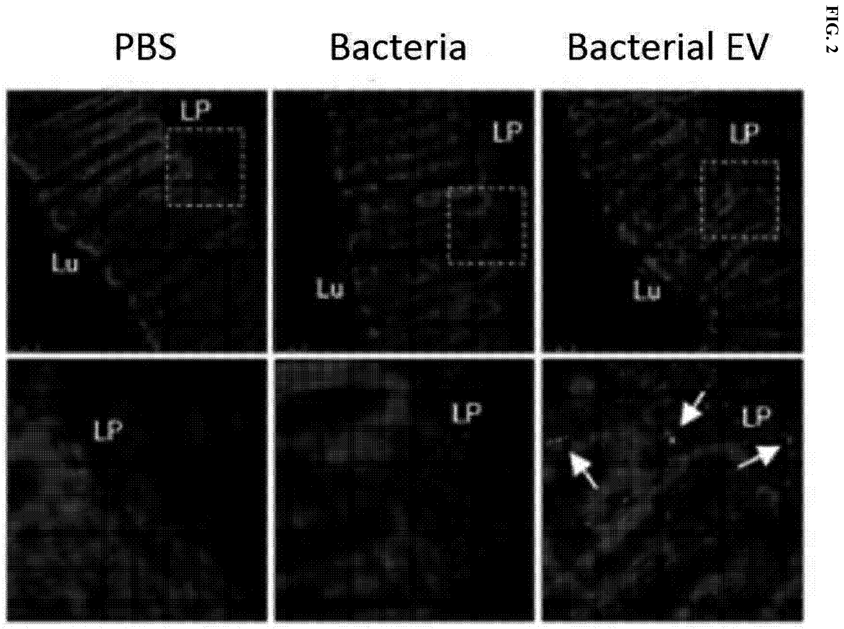

As a result, it was confirmed that bacteria failed to pass through the protective membrane of the mucosa, whereas vesicles derived from bacteria passed through the mucosa and infiltrated into the epithelial tissues (see FIG. 2).

### Example 3. Metagenomic Analysis of Vesicles Derived from Bacteria present in Clinical Sample

After blood, a nasal mucosal tissue, or the like was first placed in a 10 ml tube, suspended matter was allowed to settle by a centrifuge (3,500 x g, 10 min, 4°C), and then only the supernatant was transferred to a new 10-ml tube. After bacteria and impurities were removed by using a 0.22-µm filter, they were transferred to a Centriprep tube (centrifugal filters 50 kD) and centrifuged at 1,500 x g and 4°C for 15 minutes, materials smaller than 50 kD were discarded, and the residue was concentrated to 10 ml. After bacteria and impurities were removed once again by using a 0.22-µm filter, the supernatant was discarded by using a ultra-high speed centrifugation at 150,000 x g and 4°C for 3 hours with a Type 90Ti rotor, and an aggregated pellet was dissolved in physiological saline (PBS).

Internal DNA was extracted out of the lipid by boiling 100 µl of the vesicles isolated by the above method at 100°C, and then cooled on ice for 5 minutes. And then, in order to remove the remaining suspended matter, the DNA was centrifuged at 10,000 x g and 4°C for 30 minutes, and only the supernatant was collected. And, the amount of DNA was quantified by using Nanodrop. Thereafter, in order to confirm whether the DNA derived from bacteria was present in the extracted DNA, PCR was performed with 16s rDNA primers shown in the following Table 1 and it was confirmed that genes derived from bacteria were present in the extracted genes.

**[Table 1]**

| primer | | Sequence | SEQ ID No. |
|---|---|---|---|
| 16S rDNA | 16S_V3_F | 5'- TCGTCGGCAGCGTCAGATGTGTATAAGA GACAGCCTACGGGNGGCWGCAG-3' | 1 |
| | 16S_V4_R | 5'- GTCTCGTGGGCTCGGAGATGTGTATAAG AGACAGGACTACHVGGGTATCTAATCC | 2 |

The DNA extracted by the above method was amplified using the 16S rDNA primers, and then sequencing was performed (Illumina MiSeq sequencer), the results were output as a standard flowgram format (SFF) file, the SFF file was converted into a sequence file (.fasta) and a nucleotide quality score file using GS FLX software (v2.9), and then the reliability estimation for the reads was confirmed, and a portion in which the window (20 bps) average base call accuracy was less than 99% (Phred score<20) was removed. For the OTU (operational taxonomy unit) analysis, clustering was performed according to sequence similarity by using UCLUST and USEARCH, the genus, family, order, class, and phylum were clustered based on 94%, 90%, 85%, 80%, and 75% sequence similarity, respectively, classification was performed at the phylum, class, order, family, and genus levels of each OTU, and bacteria having a sequence similarity of 97% or more at the genus level were profiled by using the 16S RNA sequence database (108,453 sequences) of BLASTN and GreenGenes (QIIME).

### Example 4. Metagenomic analysis of vesicles derived from bacteria in blood of patient with cirrhosis

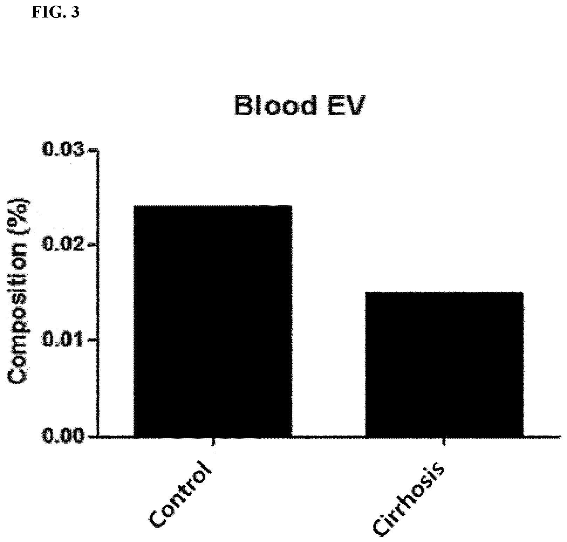

After a metagenomic analysis was performed using the method of Example 3 on the blood from 97 patients with cirrhosis and 171 normal individuals who were matched in age and sex by extracting genes from vesicles present in the blood, the distribution of vesicles derived from bacteria of the genus *Corynebacterium* was evaluated. As a result, it was confirmed that vesicles derived from bacteria of the genus *Corynebacterium* were significantly decreased in the blood from the patients with cirrhosis and liver cancer as compared to the blood from the normal individuals (see FIG. 3).

### Example 5. Metagenomic analysis of vesicles derived from bacteria in blood of patient with stroke

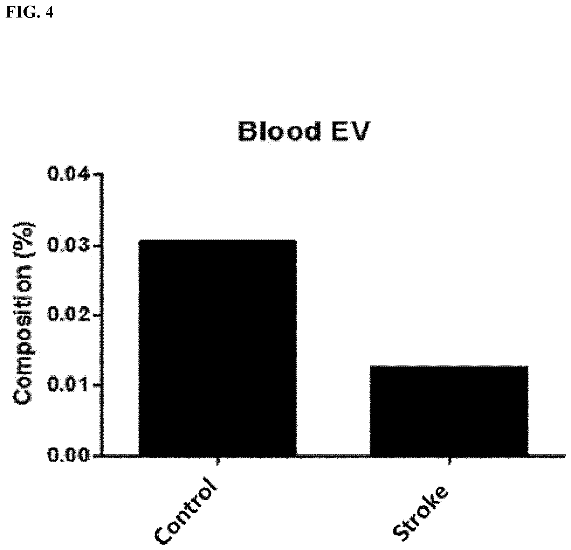

After a metagenomic analysis was performed using the method of Example 3 on the blood from 79 patients with stroke and 158 normal individuals who were matched in age and sex by extracting genes from vesicles present in the blood, the distribution of vesicles derived from bacteria of the genus *Corynebacterium* was evaluated. As a result, it was confirmed that vesicles derived from bacteria of the genus *Corynebacterium* were significantly decreased in the blood from the patients with stroke as compared to the blood from the normal individuals (see FIG. 4).

### Example 6. Metagenomic analysis of vesicles derived from bacteria in blood of patient with diabetes

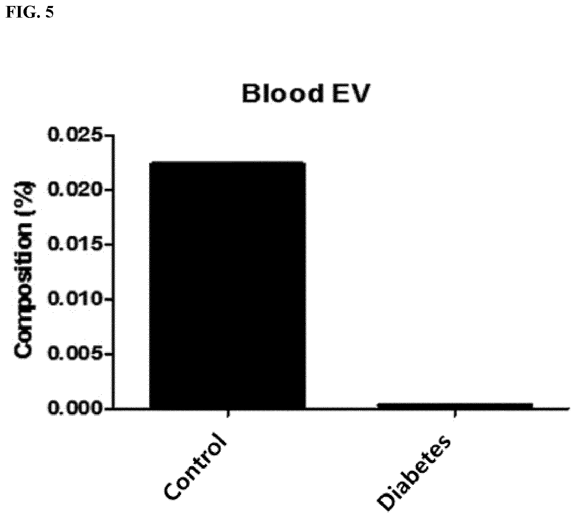

After a metagenomic analysis was performed using the method of Example 3 on the blood from 81 patients with diabetes and 126 normal individuals who were matched in age and sex by extracting genes from vesicles present in the blood, the distribution of vesicles derived from bacteria of the genus *Corynebacterium* was evaluated. As a result, it was confirmed that vesicles derived from bacteria of the genus *Corynebacterium* were significantly decreased in the blood from the patients with diabetes as compared to the blood from the normal individuals (see FIG. 5).

### Example 7. Metagenomic analysis of vesicles derived from bacteria in blood of patient with asthma

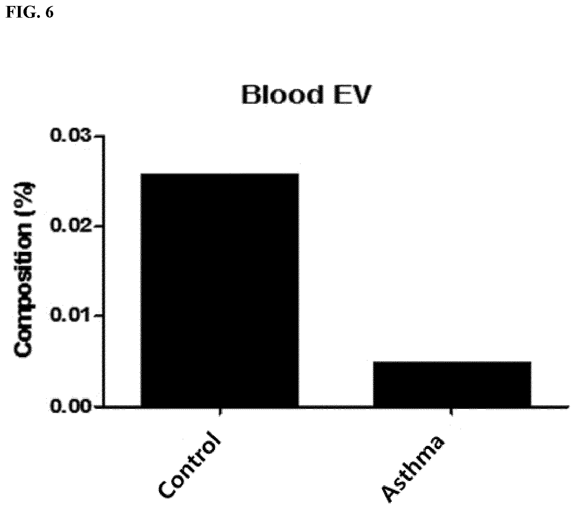

After a metagenomic analysis was performed using the method of Example 3 on the blood from 182 patients with asthma and 180 normal individuals who were matched in age and sex by extracting genes from vesicles present in the blood, the distribution of vesicles derived from bacteria of the genus *Corynebacterium* was evaluated. As a result, it was confirmed that vesicles derived from bacteria of the genus *Corynebacterium* were significantly decreased in the blood from the patients with asthma as compared to the blood from the normal individuals (see FIG. 6).

### Example 8. Metagenomic analysis of vesicles derived from bacteria in blood of patient with atopic dermatitis

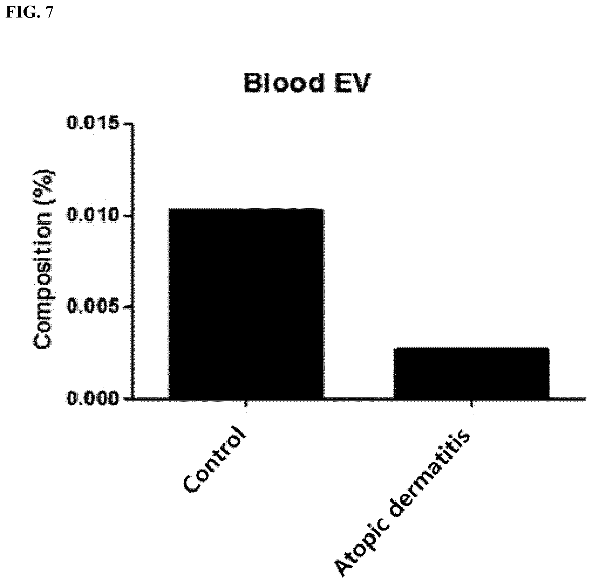

After a metagenomic analysis was performed using the method of Example 3 on the blood from 42 patients with atopic dermatitis and 40 normal individuals who were matched in age and sex by extracting genes from vesicles present in the blood, the distribution of vesicles derived from bacteria of the genus *Corynebacterium* was evaluated. As a result, it was confirmed that vesicles derived from bacteria of the genus *Corynebacterium* were significantly decreased in the blood from the patients with atopic dermatitis as compared to the blood from the normal individuals (see FIG. 7).

### Example 9. Metagenomic analysis of vesicles derived from bacteria in blood of patient with depression

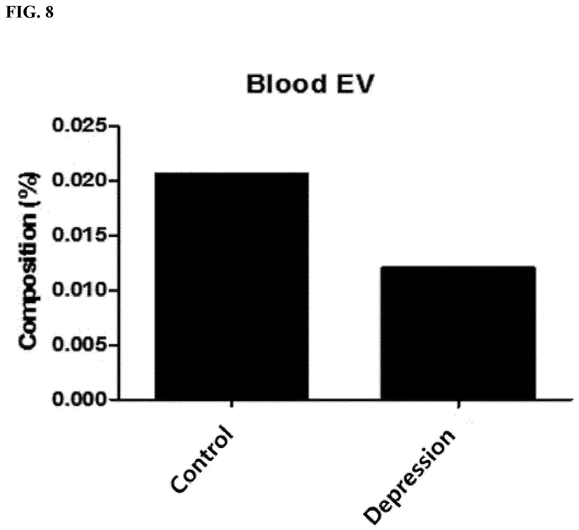

After a metagenomic analysis was performed using the method of Example 3 on the blood from 72 patients with depression and 80 normal individuals who were matched in age and sex by extracting genes from vesicles present in the blood, the distribution of vesicles derived from bacteria of the genus *Corynebacterium* was evaluated. As a result, it was confirmed that vesicles derived from bacteria of the genus *Corynebacterium* were significantly decreased in the blood from the patients with depression as compared to the blood from the normal individuals (see FIG. 8).

### Example 10. Metagenomic analysis of vesicles derived from bacteria in blood of patient with breast cancer

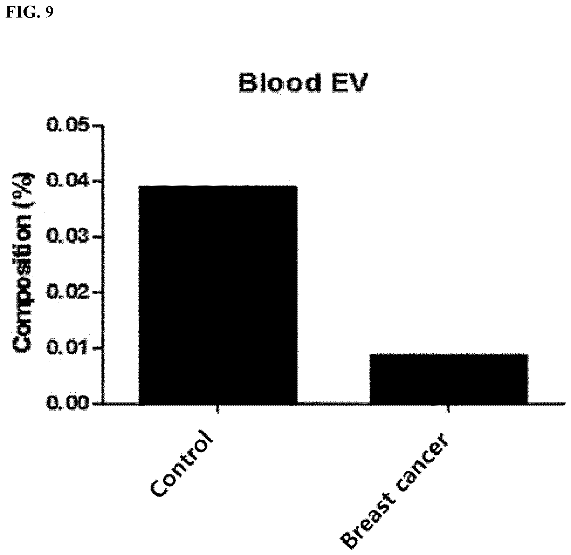

After a metagenomic analysis was performed using the method of Example 3 on the blood from 102 patients with breast cancer and 100 normal individuals who were matched in age and sex by extracting genes from vesicles present in the blood, the distribution of vesicles derived from bacteria of the genus *Corynebacterium* was evaluated. As a result, it was confirmed that vesicles derived from bacteria of the genus *Corynebacterium* were significantly decreased in the blood from the patients with breast cancer as compared to the blood from the normal individuals (see FIG. 9).

### Example 11. Metagenomic analysis of vesicles derived from bacteria in blood of patient with dementia

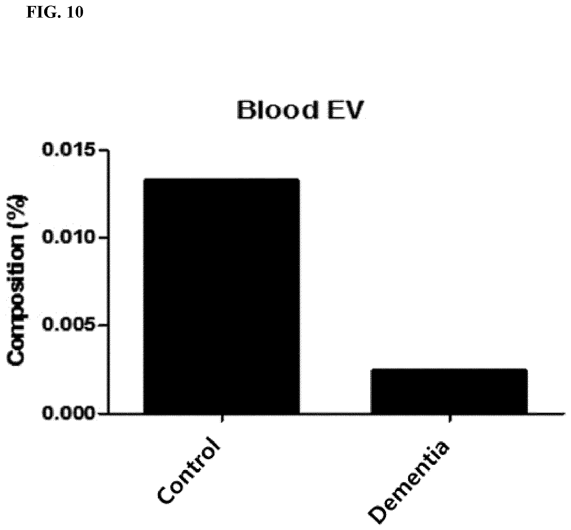

After a metagenomic analysis was performed using the method of Example 3 on the blood from 73 patients with dementia and 70 normal individuals who were matched in age and sex by extracting genes from vesicles present in the blood, the distribution of vesicles derived from bacteria of the genus *Corynebacterium* was evaluated. As a result, it was confirmed that vesicles derived from bacteria of the genus *Corynebacterium* were significantly decreased in the blood from the patients with dementia as compared to the blood from the normal individuals (see FIG. 10).

### Example 12. Infiltration patterns of vesicles derived from bacteria of the genus Corynebacterium through nasal mucosal metagenomic analysis of patient with nasal polyps and normal control

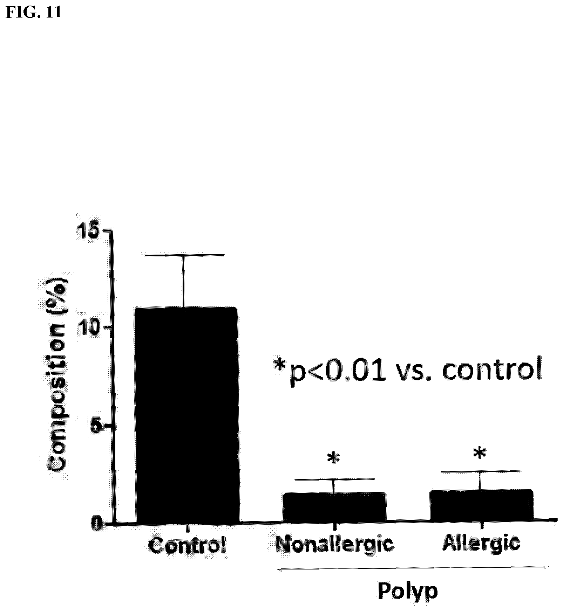

A metagenomic analysis was performed to evaluate the infiltration patterns of vesicles derived from bacteria in the nasal mucosa of patients with nasal polyps (43 nonallergic patients and 45 allergic patients) and a normal control of 39 persons by the method in Example 3. As a result, in the nasal tissues of the patients with allergic nasal polyps and the patients with nonallergic nasal polyps, vesicles derived from bacteria of the genus *Corynebacterium* were significantly reduced compared with the normal control, and there was no difference in the presence or absence of allergic polyps and the degree of infiltration of vesicles derived from bacteria of the genus *Corynebacterium* among the patients with polyps (see FIG. 11).

### Example 13. Isolation of Vesicles from Corynebacterium glutamicum Culturing Solution

Based on the above examples, a *Corynebacterium glutamicum* strain were cultured, and then vesicles were isolated therefrom and characteristics of the isolated vesicles were analyzed. First, the *Corynebacterium glutamicum* strains were cultured in a de Man-Rogosa and Sharpe (MRS) medium in an incubator at 37°C until the absorbance (OD 600) became 1.0 to 1.5, and then sub-cultured in a Luria-Bertani (LB) medium. Subsequently, a culture supernatant including the strain was recovered and centrifuged at 10,000 x g and 4 °C for 20 minutes, and then the strain was removed and filtered through a 0.22 µm filter.

The filtered supernatant was concentrated to a volume of less than or equal to 50 ml through microfiltration by using a MasterFlex pump system (Cole-Parmer, US) with a 100 kDa Pellicon 2 Cassette filter membrane (Merck Millipore, US), and the concentrated supernatant was filtered once again with a 0.22-µm filter. Thereafter, proteins were quantified by using a BCA assay, and the following experiments were performed on the obtained vesicles.

### Example 14. Inflammation-inducing Effect of vesicles derived from Corynebacterium glutamicum

To examine an effect of vesicles derived from *Corynebacterium glutamicum* (*Corynebacterium glutamicum* EV, SPC101) on the secretion of inflammatory mediators (IL-6 and TNF-α) in inflammatory cells, Raw 264.7 cells, which is a mouse macrophage line, were treated with vesicles derived from *Corynebacterium glutamicum* at various concentrations (0.1, 1, or 10 µg/ml), followed by apoptosis and ELISA.

More specifically, Raw 264.7 cells aliquoted at 5 x 10⁴ cells/well into a 48-well cell culture plate were treated with vesicles derived from *Corynebacterium glutamicum* at various concentrations, which were diluted with a DMEM (Dulbecos Modified Eagles Medium) serum-free medium, and the treated cells were cultured for 12 hours. Thereafter, the cell culture solution was collected in a 1.5-ml tube and centrifuged at 3,000 g for 5 minutes, the supernatant was recovered and stored at -80°C, and then an ELISA analysis was performed.

For ELISA, a capture antibody was diluted with phosphate buffered saline (PBS) and 50 µl aliquots thereof were dispensed into a 96-well polystyrene plate in accordance with a working concentration, and then allowed to react at 4 °C overnight. Subsequently, the sample was washed three times with 100 µl of a PBST (0.05% Tween-20-containing PBS) solution, and then an RD (1% bovine serum albumin (BSA)-containing PBS) solution was dispensed in 100 µl aliquots, followed by blocking at room temperature for 1 hour, and then the sample and a standard were dispensed in 50 µl aliquots in accordance with concentration and allowed to react at room temperature for 2 hours. Then, the sample and the standard were washed three times with 100 µl of PBST, and then the detection antibody was diluted with RD, and the diluted solution was dispensed in 50 µl aliquots in accordance with a working concentration and allowed to react at room temperature for 2 hours. Thereafter, the sample and the standard were washed three times with 100 µl of PBST, and then streptavidin-horseradish peroxidase (HRP) (R&D Systems, USA) was diluted in RD to 1/40, and the diluted solution was dispensed in 50 µl aliquots and allowed to react at room temperature for 20 minutes. Lastly, the sample and the standard were washed three times with 100 µl of PBST, and then a 3,3',5,5'-tetramethylbenzidine (TMB) substrate (SurModics, USA) was dispensed in 50 µl aliquots, and then when color was developed after 5 minutes to 20 minutes, a 1M sulfuric acid solution was dispensed in 50 µl aliquots, thereby stopping the reaction, and absorbance at 450 nm was measured using a SpectraMax M3 microplate reader (Molecular Devices, USA).

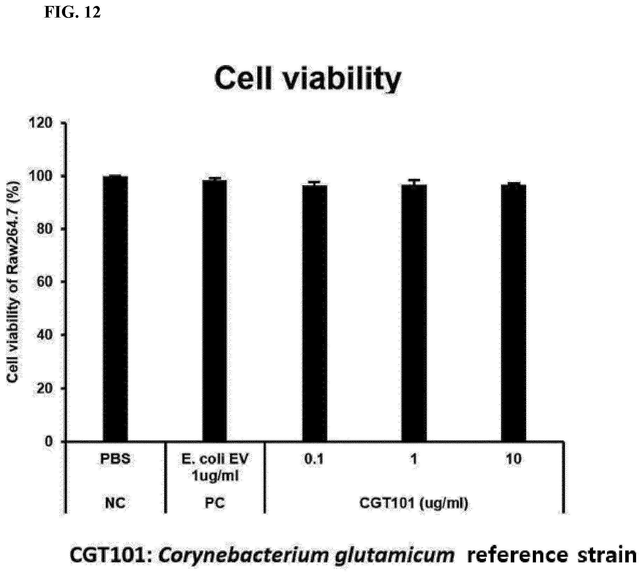

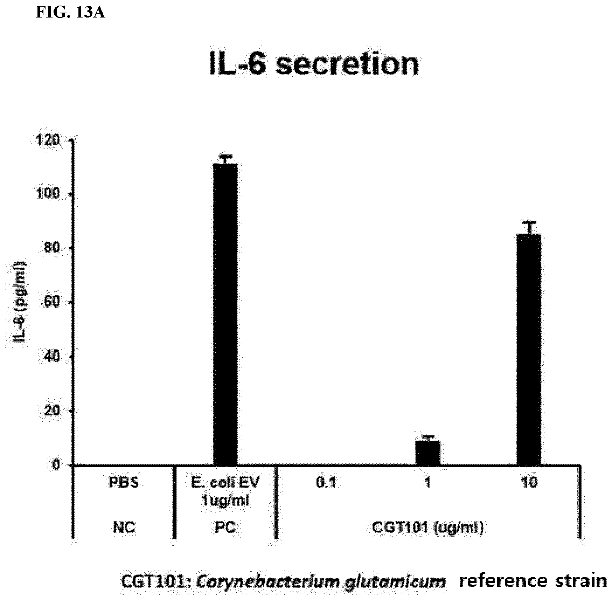

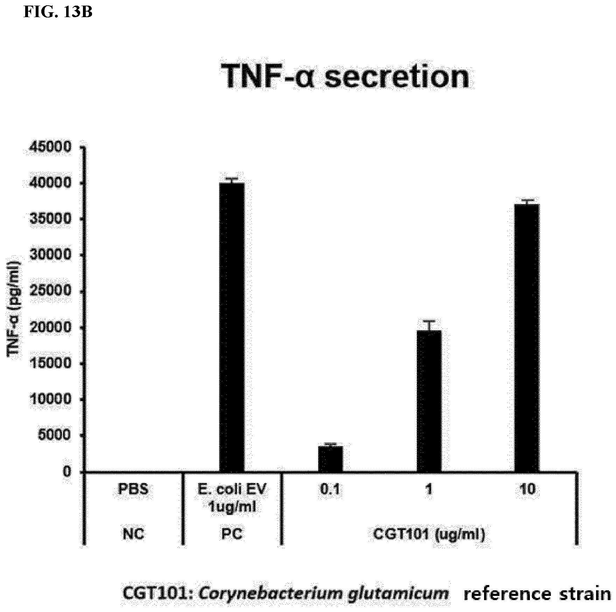

As a result, as illustrated in FIG. 12, apoptosis due to the treatment with vesicles (CGT 101) derived from *Corynebacterium glutamicum* was not observed (see FIG. 12). Further, as illustrated in FIGS. 13A and 13B, as a result of evaluating the secretion pattern of inflammatory mediators in inflammatory cells, it was confirmed that the secretion of IL-6 (FIG. 13A) and TNF-α (FIG. 13B) was much reduced upon treatment with vesicles (CGT 101) derived from *Corynebacterium glutamicum* compared to upon treatment with E. coli-derived vesicles (E. *coli* EV 1 µg/ml), which are a positive control.

### Example 15. Anti-inflammatory Effects of Vesicles derived from Corynebacterium glutamicum

In order to evaluate the anti-inflammatory effects of vesicles derived from *Corynebacterium glutamicum* based on the result of Example 14, after mouse macrophage cell lines were pre-treated with vesicles (CGT 101) derived from *Corynebacterium glutamicum* at various concentrations (0.1, 1, and 10 µg/ml) for 12 hours, the cell lines were treated with 1 µg/ml of *E. coli*-derived vesicles, which are a pathogenic factor, and then the secretion of inflammatory cytokines was measured by ELISA after 12 hours.

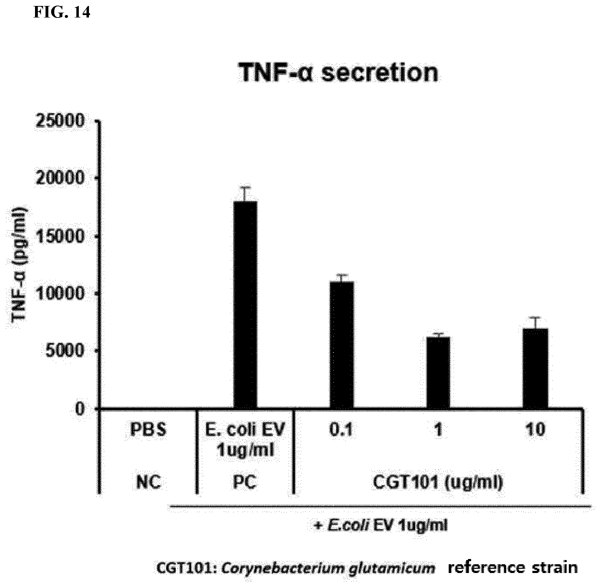

As a result, it was found that the amount of TNF-α secreted into inflammatory cells by *E. coli* EV stimulation during pre-treatment of vesicles derived from *Corynebacterium glutamicum* was remarkably suppressed (see FIG. 14). This means that vesicles derived from *Corynebacterium glutamicum* can efficiently suppress inflammatory responses induced by proinflammatory factors such as vesicles derived from *E coli*, EVs which are pathogenic vesicles.

The above-described description of the present invention is provided for illustrative purposes, and those of ordinary skill in the art to which the present invention pertains will understand that the present invention can be easily modified into other specific forms without changing the technical spirit or essential features of the present invention. Therefore, it should be understood that the above-described Examples are illustrative only in all aspects and are not restrictive.

### [industrial Applicability]

Since it was confirmed that vesicles derived from bacteria of the genus *Corynebacterium* according to the present invention passed through the protective membrane of the mucosa, and were absorbed by the mucosal epithelial cells, systemically distributed, and excreted from the body through the kidneys, liver, and lungs, it was confirmed that the vesicles were significantly reduced in the blood or nasal mucosa of patients with cirrhosis, stroke, diabetes, asthma, atopic dermatitis, depression, breast cancer, dementia, and nasal polyps, and it is possible to significantly suppress the secretion of inflammatory mediators such as TNF-α from pathogenic vesicles, the vesicles derived from bacteric of the genus *Corynebacterium* are expected to have great industrial utility value in that the vesicles are usefully used in a composition for prevention or treatment of inflammatory diseases.

## Claims

1. A method of providing information for diagnosing one or more diseases selected from the group consisting of cirrhosis, stroke, diabetes, asthma, atopic dermatitis, depression, breast cancer, dementia, and nasal polyps, the method comprising the following steps:
(a) extracting DNAs from vesicles isolated from samples of a normal individual and a subj ect;
(b) performing polymerase chain reaction (PCR) on the extracted DNA using a pair of primers prepared based on a gene sequence present in 16S rDNA to obtain each PCR product; and
(c) determining a case in which a content of vesicles derived from bacteria of the genus *Cornynebacterium* is lower than that of the normal individual sample, as one or more diseases selected from the group consisting of cirrhosis, stroke, diabetes, asthma, atopic dermatitis, depression, breast cancer, dementia, and nasal polyps, through quantitative analysis of the PCR product.

2. The method of claim 1, wherein the sample in Step (a) is blood, urine, stool, saliva, or nasal mucosa.

3. A pharmaceutical composition for preventing or treating an inflammatory disease, comprising vesicles derived from bacteria of the genus *Cornynebacterium* as an active ingredient.

4. The pharmaceutical composition of claim 3, wherein the vesicles derived from bacteria of the genus *Cornynebacterium* are secreted from a *Cornynebacterium glutamicum*.

5. The pharmaceutical composition of claim 3, wherein the vesicles have an average diameter of 10 to 200 nm.

6. The pharmaceutical composition of claim 3, wherein the vesicles are secreted naturally or artificially from bacteria of the genus *Cornynebacterium.*

7. The pharmaceutical composition of claim 3, whererin the inflammatory disease is one or more diseases selected from the group consisting of cirrhosis, stroke, diabetes, asthma, atopic dermatitis, depression, breast cancer, dementia, and nasal polyps.

8. A food composition for preventing or alleviating an inflammatory disease, comprising vesicles derived from bacteria of the genus *Cornynebacterium* as an active ingredient.

9. The food composition of claim 8, wherein the vesicles derived from bacteria of the genus *Cornynebacterium* are secreted from a *Cornynebacterium glutamicum.*

10. The food composition of claim 8, wherein the vesicles have an average diameter of 10 to 200 nm.

11. The food composition of claim 8, wherein the vesicles are secreted naturally or artificially from bacteria of the genus *Cornynebacterium.*

12. The food composition of claim 8, whererin the inflammatory disease is one or more diseases selected from the group consisting of cirrhosis, stroke, diabetes, asthma, atopic dermatitis, depression, breast cancer, dementia, and nasal polyps.

13. A cosmetic composition for preventing or alleviating an inflammatory disease, comprising vesicles derived from bacteria of the genus *Cornynebacterium* as an active ingredient.

14. An inhalable composition for preventing or treating an inflammatory disease, comprising vesicles derived from bacteria of the genus *Cornynebacterium* as an active ingredient.

15. A method of diagnosing one or more diseases selected from the group consisting of cirrhosis, stroke, diabetes, asthma, atopic dermatitis, depression, breast cancer, dementia, and nasal polyps, the method comprising the following steps:
(a) extracting DNAs from extracellular vesicles isolated from samples of a normal individual and a subject;
(b) performing polymerase chain reaction (PCR) on the extracted DNA using a pair of primers prepared based on a gene sequence present in 16S rDNA to obtain each PCR product; and
(c) determining a case in which a content of vesicles derived from bacteria of the genus *Cornynebacterium* is lower than that of the normal individual sample, as one or more diseases selected from the group consisting of cirrhosis, stroke, diabetes, asthma, atopic dermatitis, depression, breast cancer, dementia, and nasal polyps, through quantitative analysis of the PCR product.

16. A method of preventing or treating an inflammatory disease, the method comprising a step of administering a composition comprising vesicles derived from bacteria of the genus *Cornynebacterium* as an active ingredient to a subject.

17. A use of vesicles derived from bacteria of the genus *Cornynebacterium* for preparing a drug used for an inflammatory disease.


<110>    MD Healthcare Inc.

<120>    Nanovesicles derived from Corynebacterium bacteria and Use
         thereof

<130>    MPCT19-135

<150>    KR 10-2018-0158622
<151>    2018-12-10

<150>    KR 10-2019-0132137
<151>    2019-10-23

<160>    2

<170>    KoPatentIn 3.0

<210>    1
<211>    50
<212>    DNA
<213>    Artificial Sequence

<220>
<223>    16S_V3_F


<400>    1
tcgtcggcag cgtcagatgt gtataagaga cagcctacgg gnggcwgcag                50


<210>    2
<211>    55
<212>    DNA
<213>    Artificial Sequence

<220>
<223>    16S_V4_R


<400>    2
gtctcgtggg ctcggagatg tgtataagag acaggactac hvgggtatct aatcc          55